# EUROPÄISCHE PATENTSCHRIFT

(11) **EP 0 946 709 B1**
(45) Veröffentlichungstag und Bekanntmachung des Hinweises auf die Patenterteilung: **05.06.2002**
(21) Anmeldenummer: 97952938.5
(22) Anmeldetag: 12.12.1997
(51) Int. Cl.: C12M 1/42, G01N 33/487, B03C 5/02, H02N 13/00

(54) **ELEKTRODENANORDNUNG FÜR FELDKÄFIGE**
ELECTRODE ARRAY FOR FIELD CAGES
GROUPEMENT D'ELECTRODES POUR CAGES DE CHAMPS

(30) Priorität: 20.12.1996 DE 19653659
(43) Veröffentlichungstag der Anmeldung: 06.10.1999
(73) Patentinhaber: Evotec OAI AG, 22525 Hamburg (DE)
(72) Erfinder: FUHR, Günter, D-22525 Hamburg (DE); SCHNELLE, Thomas, D-10243 Berlin (DE); FIEDLER, Stefan, D-10119 Berlin (DE); SHIRLEY, Stephen, Graham, Brandon, Warks CV8 3HW (GB)
(74) Vertreter: Hertz, Oliver, Dr.
(86) Internationale Anmeldenummer: EP9707002
(87) Internationale Veröffentlichungsnummer: WO9828405

(56) Entgegenhaltungen:
- DE-A- 19 500 660
- FUHR G ET AL: "POSITIONING AND MANIPULATION OF CELLS AND MICROPARTICLES USING MINIATURIZED ELECTRIC FIELD TRAPS AND TRAVELLING WAVES" SENSORS AND MATERIALS, Bd. 7, Nr. 2, 1995, TOKYO JP, Seiten 131-146, XP000617993

## Beschreibung

Die Erfindung betrifft Elektrodenanordnungen zur Bildung von Feldkäfigen, die insbesondere in Mikrosystemen zur Handhabung und Manipulierung von Teilchen eingesetzt werden, und Feldkäfigelektroden zur Verwendung in derartigen Elektrodenanordnungen.

Bei zahlreichen biotechnologischen, medizinischen, gentechnischen und chemisch-technologischen Systemen ist es für die Erzielung bestimmter Zustände oder für den Ablauf bestimmter Prozesse erforderlich, daß mikroskopisch kleine Teilchen in freien Flüssigkeiten präzise gehaltert oder in vorbestimmter Weise bewegt werden. Zu den interessierenden kleinen Teilchen zählen z.B. biologische Zellen oder Teile von diesen, Latexpartikeln oder andere sogenannte Microbeads. Die Teilchen werden unter dem Einfluß elektrischer Felder in sogenannten Feldkäfigen in der Flüssigkeit bewegt ("offene" Feldkäfige) oder gehaltert ("geschlossene Feldkäfige"). Im gehalterten Zustand können die Teilchen beispielsweise vermessen oder untereinander zur Wechselwirkung gebracht werden (s. z.B. U. Zimmermann et al. "Electromanipulation of Cells" in CRC, Chapter 5, Seiten 259-328, 1996).

Die Felder zur Beeinflussung der Teilchen werden durch Elektroden gebildet, die vorzugsweise mit den Methoden der Halbleitertechnologie gefertigt werden. Zur Bildung von Feldkäfigen wird eine Mehrzahl von Elektroden entweder zwei- oder dreidimensional so angeordnet und mit Potentialen beaufschlagt, daß im von den Elektroden umgebenen Raum (sogenannter Innenraum) eine Feldverteilung gebildet wird, in der ein Teilchen gefangen (getrapt) oder gerichtet bewegt werden kann.

Die Elektrodenform und -anordnungen für Mikrosystemanwendungen wurden bislang lediglich in Bezug auf die Effektivität der Käfigbildung im Innenraum optimiert (s. S. Fiedler et al. in "Microsystem Technology" 2, 1-7, 1995, oder G. Fuhr et al. in "Sensors and Materials", 7, 131, 1995). Es ist beispielsweise bekannt, bei Feldkäfigen einer Größe von einigen Mikrometern bis zu einigen 100 Mikrometern Elektroden mit einer hyperbolisch, sich zum Innenraum hin verjüngenden Streifenform auszubilden. Derartige einfache Elektrodenformen wurden bisher für optimal in Bezug auf die Feldkäfigbildung und in Bezug auf deren Optimierung durch eine verhältnismäßig einfache Berechenbarkeit der Feldgradienten betrachtet. Allerdings tritt bei den herkömmlichen Elektrodenanordnungen und -formen in nachteiliger Weise der folgende Effekt -auf.

Bei geringen Teilchendichten in der Flüssigkeit (Suspension) können einzelne Teilchen (z.B. eine Zelle) über lange Zeit (Minuten bis Stunden) stabil im Feldkäfig gehalten werden. Bei höheren Teilchendichten hingegen treten im Lauf der Zeit weitere Teilchen in den Feldkäfig ein, was in der Regel unerwünscht ist. Diese Erscheinung ist zunächst überraschend, da der Käfig einen Potentialwall darstellt, der ein Einwandern von Teilchen in das Innere verhindern sollte. Es treten jedoch zusätzliche, zum Innenraum hin gerichtete Kräfte auf, die es den Teilchen erlauben, das Potential zu überwinden. Diese Kräfte werden durch eine Thermokonvektion (insbesondere Erwärmung in Elektrodennähe) verursacht.

Aufgrund dieser Erscheinung ist der Einsatzbereich von Feldkäfigen in Mikrosystemen auf verhältnismäßig geringe, in der Praxis häufig nicht akzeptable Teilchendichten beschränkt.

Die Aufgabe der Erfindung ist es, eine verbesserte Elektrodenanordnung zur Käfigbildung und verbesserte Feldkäfigelektroden für eine derartige Elektrodenanordnung anzugeben, mit denen größere Teilchenkonzentrationen als mit den herkömmlichen Mikrosystemen mit hoher Stabilität und Zuverlässigkeit gehandhabt bzw. bearbeitet werden können.

Diese Aufgabe wird durch eine Elektrodenanordnung bzw. eine Feldkäfigelektrode mit den Merkmalen der Patentansprüche 1 bzw.9 gelöst. Vorteilhafte Ausführungsformen der Erfindung ergeben sich aus den abhängigen Ansprüchen.

Die Erfindung basiert auf der Idee, neue Elektrodenformen und -anordnungen zu schaffen, mit denen einerseits thermokonvektive Strömungen vermindert oder unterdrückt und andererseits die Wirksamkeit des den Feldkäfig umgebenden Potentialwalls erhöht wird. Erfindungsgemäße Elektrodenanordnungen bestehen aus einer Mehrzahl von Feldkäfigelektroden, die jeweils einen End- oder Kopfbereich aufweisen, der über einen Zuleitungsbereich mit einem elektrischen Potential beaufschlagbar ist. Abweichend von den herkömmlichen Elektrodenformen mit glatten, stetig verlaufenden Rändern werden erfindungsgemäß die Endbereiche von Elektroden derart geformt, daß sowohl im Innenraum (Feldkäfig) als auch im Außenraum stark inhomogene Feldverläufe entstehen. Dabei ist Inhomogenität der Feldverläufe so stark bzw. sind die Feldgradienten so groß, daß außerhalb des Feldkäfigs ein Abschirmfeld gebildet wird. Das Abschirmfeld soll mindestens so stark sein, daß Kräfte auf die Teilchen wirken, die nach innen gerichtete Kräfte (z.B. durch Thermokonvektion) kompensieren. Zu diesem Zweck weist der Endbereich einer Elektrode Elektrodenabschnitte auf, die zumindest teilweise durch gerade oder schwach gekrümmte Ränder begrenzt sind, die unter vorbestimmten Winkeln aufeinanderstoßen. Die Ränder stoßen so aufeinander, daß eine unstetige Begrenzung (Bildung einer Ecke oder Spitze oder dgl.) entsteht. Damit werden an den Rändern des Endbereiches hohe Feldlinienkonzentrationen erzielt, die die gewünschten großen Feldgradienten ergeben.

Gemäß einer bevorzugten Ausführungsform der Erfindung wird neben der genannten Formung des Endbereiches zur Bildung inhomogener Abschirmfelder der Zuleitungsbereich, über den der Endbereich mit einem Elektrodenanschluß verbunden ist, mit einer möglichst geringen Elektrodenoberfläche ausgeführt.

Vorzugsweise besitzt der Zuleitungsbereich eine Streifenform mit einer unter Berücksichtigung der elektrischen Leistung optimierten Streifenbreite. Die Gestaltung möglichst schmaler Zuleitungsbereiche führt zur Verringerung von Thermokonvektionen.

Erfindungsgemäß wird von den bisher verwendeten, mit Blick auf die Feldkäfigbildung optimierten Elektrodenformen abgewichen und stattdessen werden Elektroden bereitgestellt, deren Endbereiche die Bildung von Feldlinienkonzentrationen z.B. an Kanten oder Spitzen der Elektrodenränder erlauben.

Erfindungsgemäße Elektrodenanordnungen können im wesentlichen planar zweidimensional angeordnet sein, wobei dann die Teilchenhalterung oder -bewegung unter Zusammenwirkung des Feldkäfigs mit Teilen des Mikrosystems (mechanische Begrenzung) erfolgt. Ersatzweise können die Elektrodenanordnungen auch dreidimensionale Gebilde sein, in denen die Teilchen lediglich unter Wirkung der elektrischen Kräfte in den Feldkäfigen gehalten werden. Aber auch im Fall dreidimensionaler Systeme können mechanische Begrenzungen zum Zusammenwirken mit den Feldkäfigen vorgesehen sein.

Gemäß einer besonderen Ausführungsform der Erfindung werden nicht nur die Endbereiche der Elektroden, sondern auch die Zuleitungsbereiche mit Elektrodenabschnitten versehen, die die Bildung starker Feldgradienten ermöglichen. Dies erlaubt insbesondere bei geeignet gestaltetem Zusammenwirken von benachbart angeordneten Feldkäfigelektroden, daß offene und geschlossene Feldkäfige z.B. in Form eines Feldkäfigs mit Zuführungskanal kombiniert werden.

Erfindungsgemäße Verfahren und Vorrichtungen können vorzugsweise für die Korrelationspektroskopie, insbesondere zum Nachweis fluoreszierender Moleküle an der Oberfläche von Submikrometer- oder Mikrometer-Teilchen und/oder -Zellen, oder für pharmakologische, medizinisch-diagnostische und/oder evolutions-biotechnologische Anwendungen benutzt werden. Insbesondere können als Nachweisverfahren die Methode der Fluoreszenz-Korrelationsspektroskopie (WO 94/16313) sowie andere, insbesondere konfokale Fluoreszenztechniken, wie in der Offenlegungsschrift WO 96/13744 und der europäischen Patentanmeldung 96116373.0 vorgeschlagen, eingesetzt werden. Die letztgenannte Anmeldung schlägt ein Verfahren zur Analyse von Proben durch wiederholte Messung der Anzahl von Photonen pro definiertem Zeitintervall von elektromagnetischer Strahlung, insbesondere Licht, vor, welche von den Partikeln in der Probe emittiert, gestreut und/oder reflektiert wird, und Bestimmung der Verteilung der Anzahl von Photonen in den jeweiligen Zeitintervallen, wobei die Verteilung der molekularen Helligkeiten der Partikel aus der Verteilung der Anzahl von Photonen bestimmt wird.

Ausführungsformen der Erfindung werden im folgenden unter Bezug auf die beigefügten Zeichnungen beschrieben. Es zeigen:
- Fig. 1: eine schematische Draufsicht auf eine planare Elektrodenanordnung gemäß einer ersten Ausführungsform der Erfindung;
- Fig. 2: eine schematische Perspektivansicht einer räumlichen Elektrodenanordnung, die aus zwei Elektrodenanordnungen gemäß Fig. 1 besteht;
- Fign. 3A und 3B: eine weitere Ausführungsform einer erfindungsgemäßen Elektrodenanordnung sowie Gestaltungsformen von erfindungsgemäßen Feldkäfigelektroden;
- Fign. 4A und 4B: schematische Draufsichten auf Elektrodenanordnungen, bei denen erfindungsgemäß offene bzw. geschlossene Feldkäfige kombiniert sind; und
- Fig. 5: einen typischen Feldlinienverlauf.

Im folgenden werden Ausführungsbeispiele der Erfindung im einzelnen charakterisiert. Dabei ist dem Fachmann erkennbar, daß Merkmale, die in Bezug auf bestimmte Ausführungsbeispiele genannt werden, ohne weiteres auch bei anderen Ausführungsbeispielen realisierbar sind. Eine Beschränkung auf bestimmte Kombinationen von Elektrodengeometrien oder -anordnungen besteht nicht.

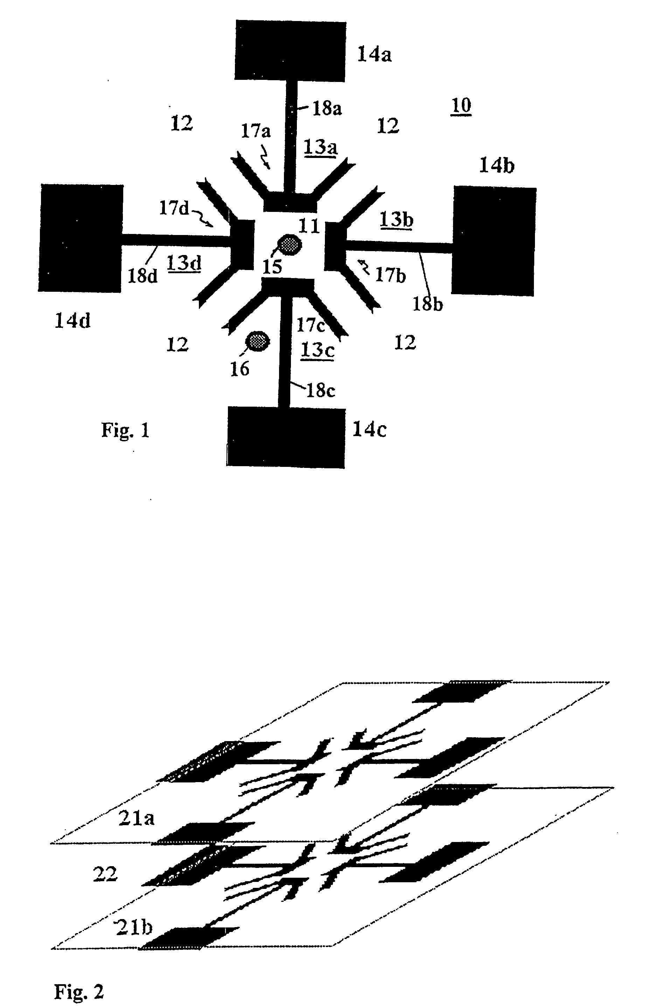

Gemäß Fig. 1 umfaßt eine erfindungsgemäße Elektrodenanordnung 10 zur Bildung eines Feldkäfigs im Innenraum 11, in dem ein Teilchen aus der umgebenden und im Innenraum befindlichen Suspension 12 gehaltert werden soll, vier Elektroden 13a-13d. Die Elektroden sind derart mit Potentialen beaufschlagbar, daß auf ein Teilchen 15 im Innenraum 11 abstoßende Polarisationskräfte (sogenannte negative Dielektrophorese) wirken, so daß das Teilchen 15 im Feldminimum gefangen wird. Ein außerhalb des Innenraumes 11 befindliches Teilchen 16 hingegen wird daran gehindert, in den Innenraum 11 einzutreten. Jede Elektrode 13a-d besteht jeweils aus einem Endbereich 17a-d, der über einen Zuleitungsbereich 18a-d mit einem Elektrodenanschluß 14a-d verbunden ist. Jeder Endbereich umfaßt streifenförmige Elektrodenabschnitte, die eine jeweils pfeilförmige Elektrodengeometrie bilden, die zum Innenraum 11 hin gerichtet ist. Die vom Innenraum weg weisenden Enden der Elektrodenabschnitte sind mit Spitzen versehen. Die dargestellte Form der Elektrodenendbereiche erlaubt die Ausbildung stark inhomogener Abschirmfelder außerhalb des Innenraums 11, d.h. in der übrigen Suspension 12. Die Zuleitungsbereiche 18a-d sind gegenüber den charakteristischen Ausmaßen der Elektrodenanschlüsse 14a-d und der Endbereiche 17a-d wesentlich verjüngt. Die Zuleitungsbereiche werden vorzugsweise durch Elektrodenstreifen gebildet, deren Breite kleiner oder gleich dem 1,5-fachen Durchmesser der zu manipulierenden Teilchen ist.

Je nach Anwendung kann die dargestellte Elektrodenanordnung eine charakteristische Größe im Bereich von 1 µm bis 500 µm oder größer besitzen. Die Elektrodenanordnung wird vorzugsweise mit planarer Halbleitertechnologie auf einem Halbleitersubstrat (Chip) gebildet und mit geeigneten Suspensionszuführungen und Abdichtungen gegenüber der Umgebung versehen.

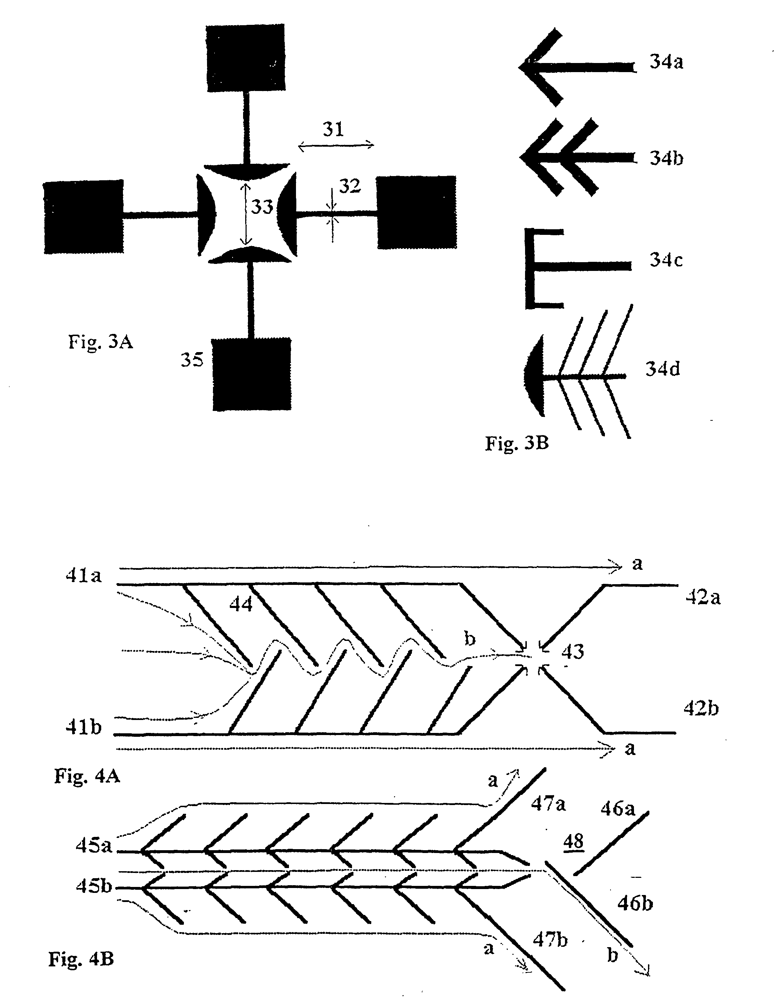

Alternativ zu der in Fig. 1 dargestellten planaren Elektrodenanordnung ist es auch möglich, einen Feldkäfig dreidimensional zu bilden. Dies wird durch eine Elektrodenanordnung gemäß Fig. 2 erreicht, die aus zwei Sub-Elektrodenanordnungen 21a, 21b besteht, die jeweils im wesentlichen einer Elektrodenanordnung gemäß Fig. 1 entsprechen können. Zwischen den Sub-Elektrodenanordnungen, die z.B. auf einem Silizium- oder Glassubstrat gebildet sind, ist eine Flüssigkeitsschicht 22 eingeschlossen, in der sich die zu manipulierenden (einzufangenden) Teilchen befinden.

Weitere Beispiele von Elektrodengestaltungen, die den Gegenstand der Erfindung verkörpern, sind in den Fig. 3A und 3B dargestellt. Die planare Elektrodenanordnung gemäß Fig. 3A entspricht im wesentlichen der Elektrodenanordnung gemäß Fig. 1, wobei jedoch die Gestalt der Elektrodenendbereiche in diesem Fall nicht pfeilförmig ist, sondern die Form eines Halbkreisabschnitts besitzt. Die Halbkreisabschnitte, die die Endbereiche bilden, erstrecken sich im wesentlichen in Richtungen, die senkrecht auf einer Verbindungslinie zwischen der Mitte des Innenraumes und dem Elektrodenanschluß liegt. Mit anderen Worten, wird der Innenraum durch Bezugsebenen von den jeweiligen Elektroden getrennt, so erstreckt sich der Endbereich jeder Elektrode im wesentlichen in einer Richtung, die in einer zu der Bezugsebene parallelen Ebene liegt.

Zur wirksamen Abhaltung von Teilchen aus dem Außenraum und zur Unterdrückung von thermokonvektiven Lösungsströmungen sollte der Abstand 31 zwischen dem Elektrodenanschluß und dem Endbereich (d.h. die Länge des Zuleitungsbereiches) mindestens dem Abstand 33 zwischen zwei gegenüberliegenden Elektroden der Elektrodenanordnung entsprechen. Ferner ist die Breite 32 des Zuleitungsbereiches vorzugsweise kleiner oder gleich 1/4 des Abstandes 33. Die Elektrodenanschlüsse, die selbst durch Zuleitungen gebildet werden, besitzen in Bezug auf ihre Breite und Länge keine Beschränkungen. Es wird jedoch bevorzugt, zur Verringerung elektrischer Verluste die Elektrodenanschlüsse 35 mit Isolationsschichten zu versehen. Die Isolationssschichten besitzen vorzugsweise eine Dicke von rund 50 nm bis einige µm.

Die schmale Ausführung des Zuleitungsbereiches besitzt den folgenden Vorteil. Eine breite Streifenelektrode bewirkt, daß ein über dieser in der Teilchensuspension befindliches Teilchen einem im wesentlichen homogenen elektrischen Feld ausgesetzt ist, in dem es leicht lateral, d.h. auch in Richtung des Feldkäfigs, durch eine Flüssigkeitsströmung verschoben werden kann. Dieser Nachteil breiter Elektrodenstreifen wird durch die erfindungsgemäße Realisierung von schmalen Elektrodenabschnitten überwunden.

Neben dem Zuleitungsbereich können auch die Endbereiche der Feldkäfigelektroden mit schmalen Streifen ausgeführt sein, wie es in Fig. 3B anhand der Endbereiche 34a-d (Pfeilform, Doppelpfeilform, T-Profil-Form, "Antennenform") beispielhaft dargestellt ist.

Die in den Fig. 1 bis 3 dargestellten Elektrodenanordnungen umfassen jeweils vier bis acht gleichartige Elektroden. Es ist jedoch auch möglich, die Erfindung mit einer anderen Anzahl von Elektroden und mit verschiedenen Elektrodengestaltungen innerhalb einer Elektrodenanordnung zu realisieren. Eine Feldkäfiganordnung in einem Mikrosystem umfaßt im weitesten Sinne (gegebenenfalls in Zusammenwirkung mit mechanischen Begrenzungsmitteln) mindestens eine Feldkäfigelektrode, mit der ein suspendiertes Teilchen manipuliert wird. Wird eine Mehrzahl von Elektroden verwendet, so sind diese möglichst symmetrisch in Bezug auf einen Innenraum, in dem der Käfio gebildet wird, angeordnet. Die Anordnung kann beispielsweise in quadratischer Form, in Form eines regelmäßigen Vielecks oder in Kreisform erfolgen. Es sind jedoch auch asymmetrische Anordnungen möglich.

In den Fig. 4A und 4B sind Mikrosysteme illustriert, die die Kombination eines Feldkäfigs 43 mit einem durch zwei Feldkäfigelektroden geformten Zuführungskanal (Fig. 4A) bzw. eine Sortiervorrichtung 48, die hier als offener Feldkäfig aufgefaßt wird, mit einem Zuführungskanal (Fig. 4B) umfaßt.'Die Fig. 4A und 4B zeigen jeweils eine Hälfte der entsprechenden Mikrosysteme. Das Gesamtsystem wird jeweils durch ein Strukturelement mit der dargestellten Elektrodenanordnung und ein zweites Strukturelement mit einer identischen, spiegelbildlich orientierten Elektrodenanordnung (unten mit einem Strich am Bezugszeichen versehen) gebildet. Beide Strukturelemente sind unter Verwendung von Abstandshaltermitteln genau ausgerichtet mit den strukturierten Oberflächen zueinanderweisend montiert. Der Zwischenraum zwischen beiden Strukturelementen wird von der Teilchensuspension durchströmt. Der Zwischenraum (d.h. der senkrechte Abstand der Elektrodenanordnungen) ist etwa so groß wie der Feldkäfig 43 breit ist.

Gemäß Fig. 4A besitzen zwei Feldkäfigelektroden des Feldkäfigs 43 eine modifizierte Gestaltung der Zuleitungsbereiche 41a und 41b, die selbst Feldkäfigelektroden für einen offenen Feldkäfig bilden. Die Zuleitungsbereiche bilden einen Zuführungskanal, über den Teilchen in vorbestimmter Weise entlang des Weges b zum Feldkäfig 43 geführt werden. Hingegen werden Teilchen auf den Wegen a am Feldkäfig 43 vorbeigeführt (hierzu können zusätzlich nicht dargestellte mechanische Trennmittel vorgesehen sein). Zur gesteuerten Führung der Teilchen zum Feldkäfig 43 sind die Zuleitungsbereiche 41a und 41b jeweils mit fischgrätenartig zur Mitte des Zuführungskanals hinweisend angeordneten Elektrodenendbereiche bildenden Streifenelektroden 44 versehen.

Die Führung der Teilchen durch den Zuführungskanal erfolgt unter vorbestimmter Ansteuerung der im Gesamtsystem zur Verfügung stehenden Zuführbereiche bzw. Elektroden 41a, 41b, 42a, 42b und den jeweiligen Gegenelektroden (nicht dargestellt) 41a', 41b', 42a' und 42b'. Alle Elektroden werden mit einem geeignet gewählten Wechselpotential der Frequenz f angesteuert, wobei zur gezielten Teilchenführung zwischen den Elektroden die folgende Phasenverschiebung realisiert wird: 41a: 0°, 41a': 180°, 41b: 180°, 41b': 0°, 42a: 180°, 42a': 0°, 42b: 0° und 42b': 180°. Die Potentialamplituden liegen vorzugsweise im Bereich 0,1 V bis 100 V und können vom Fachmann anwendungsabhängig gewählt werden.

Gemäß Fig. 4B führt ein Zuführungskanal, der durch die Elektroden 45a und 45b gebildet wird, Teilchen entlang des Weges b zu einer Sortiereinrichtung 48 mit den Elektroden 46a und 46b. Hier bildet der Zuführungskanal zur Sortiereinrichtung selbst einen offenen Feldkäfig, in dem die Elektroden mit Elektrodenabschnitten versehen sind, die zur Bildung von starken Feldinhomogenitäten eingerichtet sind. Die Elektrodenabschnitte 47a und 47b sind zusätzlich zur Ablenkung von Teilchen entlang der Wege a vorgesehen.

Zur Teilchenführung stehen wiederum vier Elektroden 45a, 45b, 46a, 46b und die entsprechenden (nicht dargestellten) Gegenelektroden 45a', 45b', 46a', 46b' zur Verfügung. Die Ansteuerung der Elektroden zur Realisierung des Pfades b erfolgt, indem die Elektroden 45a, 45a', 45b, 45b', 46b, 46b' mit Wechselpotentialen beaufschlagt werden und die Elektroden 46a und 46a' potentialfrei sind. Die eingestellte Phasenlage lautet: 45a: 0°, 45a': 180°, 45b: 180°, 45b': 0°, 46b: 0°, 46b': 180°. Falls die Teilchen nicht auf dem Pfad b (dargestellt), sondern an den Elektroden 46a, 46a' vorbeigeführt werden sollen, wären die Elektroden 46b, 46b' potentialfrei und die Elektroden 46a, 46a' mit 46a: 180°, 48a': 0° anzusteuern.

Erfindungsgemäß wird auch ein Verfahren zur Handhabung der beschriebenen Elektrodenanordnungen, insbesondere zur Einbringung von Teilchen in Feldkäfige, angegeben. Die Einführung eines oder mehrerer zur manipulierender Teilchen ist dadurch gekennzeichnet, daß eine Suspensionsströmung durch die Elektrodenanordnung geführt wird, während diese so angesteuert wird, daß mit der Suspensionsströmung geführte Teilchen aufgehalten werden. Bei einer Elektrodenanordnung gemäß Fig. 1 wird die Suspension beispielsweise in einer Richtung geführt, die bei der Darstellung von links unten nach rechts oben ausgerichtet ist. Hierzu bilden die jeweils benachbarten Elektrodenabschnitte der Elektrodenendbereiche 17d und 17c bzw. 17a und 17b (gegebenenfalls in Zusammenwirkung mit zusätzlichen, entsprechenden Elektrodenabschnitten bei einer dreidimensionalen Anordnung gemäß Fig. 2) einen Kanal, durch den die Suspension geführt wird. Während dieser Strömungsphase werden die Elektroden 13a und 13b so angesteuert, daß Teilchen, die mit der Strömung geführt werden, auf einen Potentialwall stoßen. Gleichzeitig sind die Elektroden 13c und 13d abgeschaltet. Sobald während des Durchströmschrittes, z.B. mit einer optischen Beobachtungsvorrichtung erfaßt wird, daß sich ein Teilchen im Innenraum 11 befindet, werden die Elektroden 13c und 13d zur Schließung des Feldkäfigs und zur Abschirmung weiterer. nachströmender Teilchen eingeschaltet. Ein besonderer Vorteil der erfindungsgemäßen Elektrodenanordnung und der erfindungsgemäßen Verfahrensweise besteht darin, daß diese Abtrennung gegebenenfalls nachströmender Teilchen durch die sofortige Ausbildung inhomogener Abschirmfelder sehr wirksam ist.

Typische Ansteuerprotokolle für die Manipulation von lebenden Zellen suspendiert in physiologischen Lösungen und für 3 bis 100 µm großen Beads (Latex, Sephadex etc.) sind

### Zellen: (konkret : tierische Fibroblasten, Durchmesser 15 µm)

| | |
|---|---|
| Ansteueramplituden | 2,5 bis 8 V |
| nutzbare Frequenzen | 1 MHz oder 1 GHz (üblicherweise 5 oder 10 MHz) |
| Signalform: | Sinussignale, Rechtecksignale |
| Schaltgeschwindigkeit Zu- und Abschaltung der Elektroden | 1 µs bis 1 s |
| Dauer der Feldapplikation | 1 ms bis Minuten |

Funktionsfähigkeit der Grundelemente in Abhängigkeit von der Strömungsgeschwindigkeit der Lösung im System:

| | | |
|---|---|---|
| Fischgrätensystem | (Fig. 4A, 4B) | bis zu 400 µm/s |
| Feldkäfig | (Fig. 1,2,3) | bis zu 50-100 µm/s |
| Weiche | (Fig. 4B, 46a, 46b) | bis zu 1000 µm/s |

### Mikrobeads: (konkret : Latex, Durchmeser 15: µm)

| | |
|---|---|
| Ansteueramplituden | 2,5 bis 15 V |
| nutzbare Frequenzen | 10 kHz bis 100 MHz (üblicherweise 100 bis 1 MHz) |
| Signalform | Sinussignale, Rechtecksignale |
| Schaltgeschwindigkeit Zu- und Abschaltung der Elektroden | 1 µs bis 1 s |
| Dauer der Feldapplikation | 1 ms bis Minuten |

Funktionsfähigkeit der Grundelemente in Abhängigkeit von der Strömungsgeschwindigkeit der Lösung im System:

| | | |
|---|---|---|
| Fischgrätensystem | (Fig. 4A, 4B) | bis zu 2000 µm/s |
| Feldkäfig | (Fig. 1,2,3) | bis zu 80 µm/s |
| Weiche | (Fig. 4B, 46a, 46b) | bis zu 2000 µm/s. |

Die oben unter Bezug auf Fig. 1 beispielhafte Verfahrensweise kann unter Verwendung des erläuterten Prinzips auch bei anderen Elektrodenanordnungen und -geometrien durchgeführt werden, wobei jeweils mit einer Zwei-Schritt-Prozedur die Elektroden zunächst so angesteuert werden, daß sich ein Potentialwall quer zu einer Suspensionsströmung ausbildet und sobald das oder die gewünschten Teilchen im Innenraum angelangt sind, die Elektroden so angesteuert werden, daß sich der Feldkäfig in gewünschter Weise schließt.

Die erfindungsgemäßen Elektrodenanordnungen und -verfahren können zur Vermessung, Manipulation, Konzentrierung, Aggregatbildung, Immobilisierung, Charakterisierung oder Identifizierung von Mikropartikeln, Zellen, Viren, Makromolekülen und anderen flüssigen oder festen Körpern einer Größe von 1 nm bis zu 500 µm verwendet werden.

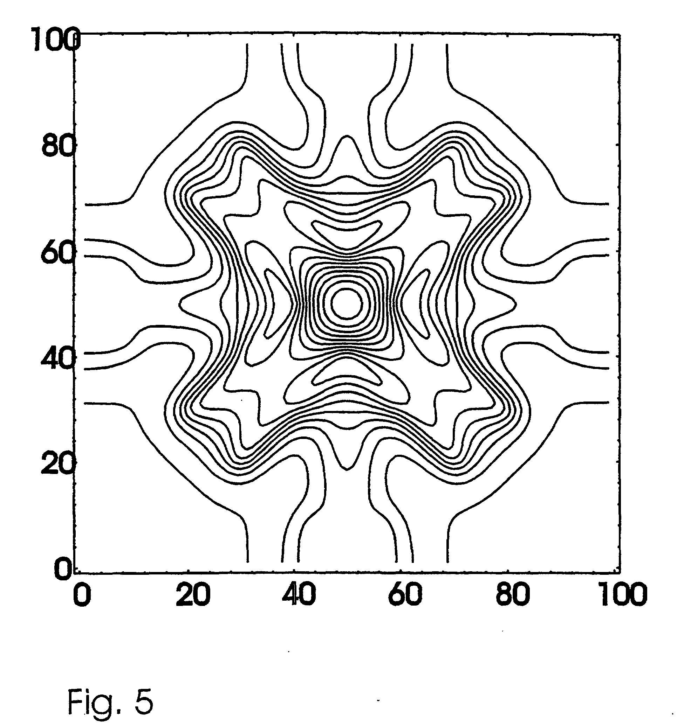

Fig. 5 zeigt einen beispielhaften Feldlinienverlauf in einem erfindungsgemäß gebildeten Feldkäfig aufgrund einer modifizierten Pfeilelektrodenanordnung. Dargestellt sind die Äquipotentiallinien (E²rms, zeitlich gemittelt, also das Potential für die dielektrophoretische Kraft). Deutlich erkennbar sind die asymmetrische Feldverteilung im Käfig wie auch außerhalb und die zugrundegelegte Pfeilelektrodenform.

## Patentansprüche

1. Elektrodenanordnung mit einer Vielzahl von Elektroden (13a-13d, 41a, 41b, 42a, 42b, 45a, 45b, 46a, 46b), die zur Bildung eines Feldkäfigs in einer Flüssigkeit eingerichtet ist, wobei die Elektroden (13a-13d, 41a, 41b, 42a, 42b, 45a, 45b, 46a, 46b) jeweils einen Endbereich (17a-17d, 34a-34d, 44) und einen Zuleitungsbereich (18a-18d) aufweisen und jeder Endbereich (17a-17d, 34a-34d, 44) über den entsprechenden Zuleitungsbereich (18a-18d) zur Bildung von Feldgradienten im Feldkäfig mit einem elektrischen Potential beaufschlagbar ist,
**dadurch gekennzeichnet, daß**
jeder Endbereich (17a-17d, 34a-34d, 44) Elektrodenabschnitte mit einer geometrischen Form aufweist, die auf der vom Feldkäfig abgewandten Seite gerade oder schwach gekrümmte Ränder umfaßt, die unter vorbestimmten Winkeln aufeinanderstoßen und eine unstetige Begrenzung bilden, wobei die Elektrodenabschnitte dazu eingerichtet sind, außerhalb des Feldkäfigs inhomogene Abschirmfelder zur bilden, die Feldgradienten aufweisen, in denen auf Teilchen (16) in der Flüssigkeit vom Feldkäfig wegweisende Kräfte wirken.

2. Elektrodenanordnung gemäß Anspruch 1, bei der die Elektrodenabschnitte eine flächige Form mit der Gestalt eines Dreiecks, Halbkreises oder T-Profils aufweisen.

3. Elektrodenanordnung gemäß Anspruch 1 oder 2, bei der die Elektrodenabschnitte durch eine Vielzahl von Streifenabschnitten gebildet sind.

4. Elektrodenanordnung gemäß einem der vorhergehenden Ansprüche, bei der sich die Elektrodenabschnitte in Richtungen erstrecken, die in einer Ebene parallel zu einer Bezugsebene zwischen dem Feldkäfig und den Endbereichen (17a-17d, 34a-34d, 44) der Elektroden (13a-13d, 41a, 41b, 42a, 42b, 45a, 45b, 46a, 46b) liegen.

5. Elektrodenanordnung gemäß einem der vorhergehenden Ansprüche, bei der jeder Zuleitungsbereich (18a-18d) durch einen Elektrodenstreifen gebildet wird, dessen Breite geringer als der 1.5-fache Durchmesser der mit der Elektrodenanordnung zu manipulierenden Teilchen (15, 16) ist.

6. Elektrodenanordnung gemäß einem der vorhergehenden Ansprüche, bei der mindestens zwei Elektroden (41a, 41b, 42a, 42b, 45a, 45b, 46a, 46b) so angeordnet sind, daß ihre Zuleitungsbereiche einen Zuführkanal bilden.

7. Elektrodenanordnung gemäß Anspruch 6, bei der die Zuleitungsbereiche mit Elektrodenendbereichen (44) verbunden sind, die ein fischgrätenartiges, in den Kanal weisendes Streifenmuster bilden.

8. Elektrodenanordnung gemäß einem der Ansprüche 1 bis 7, bei der der Feldkäfig durch einen Zuführkanal gebildet ist, der mit einer Sortiereinrichtung zusammenwirkt.

9. Feldkäfigelektrode für eine Elektrodenanordnung gemäß einem der Ansprüche 1 bis 8, die dazu eingerichtet ist, in Zusammenwirkung mit mindestens einer weiteren Feldkäfigelektrode die inhomogenen Abschirmfelder außerhalb des Feldkäfigs zu bilden.

## Claims

1. Electrode configuration with a plurality of electrodes (13a-13d, 41a, 41b, 42a, 42b, 45a, 45b, 46a, 46b) arranged to create a field cage in a liquid, whereby the electrodes (13a-13d, 41a, 41b, 42a, 42b, 45a, 45b, 46a, 46b) each have an end region (17a-17d, 34a-34d, 44) and a feed region (18a-18d) and each end region (17a-17d, 34a-34d, 44) can have an electrical potential applied to it through the corresponding feed region (18a-18d) to create field gradients in the field cage, **characterized in that** each end region (17a-17d, 34a-34d, 44) exhibits electrode segments with a geometric shape that comprises straight or slightly curved edges on the side facing away from the field cage that come up against one another at predetermined angles and form a discrete delimitation, whereby the electrode segments are adapted to form inhomogeneous shielding fields outside the field cage that exhibit field gradients in which forces away from the field cage act upon particles (16) in the liquid.

2. Electrode configuration according to claim 1 in which the electrode segments exhibit a flat form with the shape of a triangle, semicircle or T profile.

3. Electrode configuration according to claim 1 or 2 in which the electrode segments are formed by a large number of strip segments.

4. Electrode configuration according to one of the preceding claims in which the electrode segments extend in directions that are on a plane parallel to a reference plane between the field cage and the end regions (17a-17d, 34a-34d, 44) of the electrodes (13a-13d, 41a, 41b, 42a, 42b, 45a, 45b, 46a, 46b).

5. Electrode configuration according to one of the preceding claims in which each feed region (18a-18d) is formed by an electrode strip whose width is less than 1.5 times the diameter of the particles (15, 16) to be manipulated with the electrode configuration.

6. Electrode configuration according to one of the preceding claims in which at least two electrodes (41a, 41b, 42a, 42b, 45a, 45b, 46a, 46b) are arranged so that their feed regions form a feed channel.

7. Electrode configuration according to claim 6 in which the feed regions are connected to electrode end regions (44) that form a herringbone-like strip pattern pointing into the channel.

8. Electrode configuration according to one of the claims 1 through 7 in which the field cage is formed of a feed channel that acts in connection with a sorting device.

9. Field cage electrode for an electrode configuration according to one of the claims 1 through 8 that is arranged so that, interacting with at least one more field cage electrode, the inhomogeneous shielding fields are formed outside the field cage.

## Revendications

1. Disposition d'électrodes avec une multitude d'électrodes (13a - 13d, 41a, 41b, 42a, 42b, 45a, 45b, 46a, 46b), qui est agencée en vue de la formation d'une cage de champ dans un liquide, les électrodes (13a - 13d, 41a, 41b, 42a, 42b, 45a, 45b, 46a, 46b) présentant chacune une zone d'extrémité (17a - 17d, 34a - 34d, 44) et une zone d'alimentation (18a - 18d) et chaque zone d'extrémité (17a - 17d, 34a - 34d, 44) pouvant être mise sous un potentiel électrique par l'intermédiaire de la zone d'alimentation (18a - 18d) correspondante pour la formation de gradients de champ dans la cage de champ, **caractérisée en ce que** chaque zone d'extrémité(17a - 17d, 34a - 34d, 44) présente des sections d'électrode avec une forme géométrique qui comprend du côté opposé à la cage de champ des bords rectilignes ou à faible courbure, lesquels butent les uns sur les autres sous des angles prédéterminés et forment une limite discontinue, les sections d'électrode étant conçues en vue de former en dehors de la cage de champ des champs écrans hétérogènes, qui présentent des gradients de champ dans lesquels des forces agissent sur les particules (16) dans le liquide, forces tendant à les écarter de la cage de champ.

2. Disposition d'électrodes selon la revendication 1, pour laquelle les sections d'électrode présentent une forme surfacique décrivant un triangle, un demi-cercle ou un profil en T.

3. Disposition d'électrodes selon la revendication 1 ou 2, pour laquelle les sections d'électrode sont formées par une multitude de sections en bande.

4. Disposition d'électrodes selon une des revendications ci-avant, pour laquelle les sections d'électrode s'étendent dans des directions qui sont parallèles dans un plan à un plan de référence entre la cage de champ et les zones d'extrémité (17a - 17d, 34a - 34d, 44) des électrodes (13a - 13d, 41a, 41b, 42a, 42b, 45a, 45b, 46a, 46b).

5. Disposition d'électrodes selon une des revendications ci-avant, pour laquelle chaque zone d'alimentation (18a - 18d) est formée par une bande d'électrode dont la largeur est inférieure à 1,5 fois le diamètre des particules (15, 16) devant être manipulées avec la disposition d'électrodes.

6. Disposition d'électrodes selon une des revendications ci-avant, pour laquelle au moins deux électrodes (41a, 41b, 42a, 42b, 45a, 45b, 46a, 46b) sont disposées de telle sorte que leur zones d'alimentation forment un canal d'entrée.

7. Disposition d'électrodes selon la revendication 6, pour laquelle les zones d'alimentation sont reliées à des zones d'extrémité d'électrode (44) formant un motif de bandes en arêtes de poisson, orienté vers l'intérieur du canal.

8. Disposition d'électrodes selon une des revendications 1 à 7, pour laquelle la cage de champ est formée par un canal d'entrée, qui agit en synergie avec un dispositif de triage.

9. Electrode de cage de champ pour une disposition d'électrodes selon une des revendications 1 à 8, agencée en vue de former, en synergie avec au moins une autre électrode de cage de champ, les champs écrans hétérogènes à l'intérieur de la cage de champ.
